# EUROPEAN PATENT APPLICATION

(11) **EP 4 696 717 A1**
(43) Date of publication of application: **18.02.2026**
(21) Application number: 24787829.1
(22) Date of filing: 29.02.2024
(51) Int. Cl.: C07K 19/00, C07K 14/72, C12N 15/86, A61K 38/00, A61P 27/02, A61K 48/00

(54) **NEW CHIMERIC OPSIN GPCR PROTEIN FOR TREATING RETINAL NEURODEGENERATIVE DISEASES**

(30) Priority: 11.04.2023 CN 202310382404
(71) Applicant: Suzhou Ugenex Therapeutics Co., Ltd., Suzhou, Jiangsu 215000 (CN)
(72) Inventor: WEI, Xiaohu, Suzhou, Jiangsu 215000 (CN); LIN, Xi, Suzhou, Jiangsu 215000 (CN); LIU, Hongjun, Suzhou, Jiangsu 215000 (CN); CUI, Zhiping, Suzhou, Jiangsu 215000 (CN); LIU, Xiaohua, Suzhou, Jiangsu 215000 (CN); SUN, Sijie, Suzhou, Jiangsu 215000 (CN); XIE, Cheng, Suzhou, Jiangsu 215000 (CN); ZHANG, Shanzhong, Suzhou, Jiangsu 215000 (CN)
(74) Representative: Tahtadjiev, Konstantin
(86) International application number: PCT/CN2024/079320
(87) International publication number: WO 2024/212723

(57) **Abstract**

Provided are a chimeric opsin GPCR protein for treating retinal neurodegeneration diseases, the chimeric opsin GPCR protein comprising a recombinant opsin formed by replacing a corresponding domain of a natural opsin with at least one domain derived from mGluR4; a nucleic acid molecule and vector encoding the chimeric opsin; a transgenic cell comprising the chimeric opsin, the nucleic acid molecule or the vector; and the use thereof.

## Description

### Background of the Present Invention

### Field of Invention

The present application relates to the field of treatment of eye diseases. In particular, the present application relates to a GPCR chimeric opsin, comprising a recombinant opsin, wherein the recombinant opsin is formed by replacing a corresponding domain of a native opsin with at least one domain derived from mGluR4; a nucleic acid molecule and a vector encoding the chimeric opsin; a transgenic cell comprising the chimeric opsin, the nucleic acid molecule or the vector; and uses thereof.

### Description of Related Arts

Retinal neurodegenerative diseases, including blinding eye diseases such as retinitis pigmentosa (RP) and age-related macular degeneration (AMD), are caused by the degeneration and death of key retinal cells such as photoreceptor cells, which in turn leads to loss of visual function and even blindness. The pathogenic mechanism of retinal neurodegenerative diseases severely damages patients' vision, and under current therapeutic approaches, the outcome of irreversible loss of visual function remains unavoidable. The pathogenesis of retinal neurodegenerative diseases is often due to photoreceptor cell apoptosis triggered by intrinsic gene mutations, or an imbalanced retinal microenvironment (such as neovascularization or death of retinal pigment epithelial cells) that exacerbates photoreceptor apoptosis. Based on these pathogenic mechanisms, therapeutic strategies mainly focus on: (1) overexpressing or employing gene-editing to repair related mutant genes to delay photoreceptor degeneration; (2) inhibiting neovascularization to reduce damage to photoreceptors; and (3) expressing photoreceptive proteins in other retinal neurons (such as bipolar cells or ganglion cells) to perform photoreceptive functions.

The strategy of restoring vision by expressing photoreceptive proteins in other retinal neurons is currently the most actively studied. Photoreceptive proteins include endogenous photoreceptive proteins and exogenous photoreceptive proteins. In particular, the use of exogenous photoreceptive proteins, namely light-sensitive ion channel proteins (such as ChR2, ChrimsonR, MCO, etc.), has achieved vision restoration in mice, and related products have already progressed to clinical trials, with preliminary clinical results being relatively positive.

Endogenous photoreceptive proteins mainly include opsins, rhodopsin, and melanopsin. Opsins are expressed in retinal cone cells and, according to their optimal wavelength sensitivity, are classified into LW-opsins sensitive to long wavelengths, MW-opsins sensitive to medium wavelengths, and SW-opsins sensitive to short wavelengths. Rhodopsin is expressed in retinal rod cells, and melanopsin is expressed in photosensitive retinal ganglion cells. These three photoreceptive proteins all belong to the G protein-coupled receptor (GPCR) family. GPCRs are an important superfamily of membrane proteins, with more than 800 members identified to date. They are composed of a classical conserved seven-transmembrane (7TM) helical structure, three extracellular loops, three intracellular loops, an N-terminal region, and a C-terminal region. Extracellular chemical substances act as signaling molecules, i.e., ligands, which bind to GPCRs to mediate downstream intracellular signal transduction, thereby triggering a series of physiological processes. Based on sequence and structural similarity, GPCRs can be classified into five subfamilies: rhodopsin (class A), secretin (class B1), adhesion (class B2), glutamate (class C), and Taste2/Frizzled (class F). The structural characteristics and endogenous ligand-binding sites differ among families. The three endogenous photoreceptive proteins all belong to class A. For class A receptors, in most cases, endogenous ligands are recognized by the orthosteric binding site within the 7TM domain. Researchers have found that each of the three endogenous photoreceptive proteins can be applied to restore vision in blind mice, and such studies are currently in the early stages of development.

Studies have also found that fusing endogenous photoreceptive proteins with metabotropic glutamate receptors (mGluRs) can likewise partially restore vision in blind mice. mGluRs belong to class C of the G protein-coupled receptor (GPCR) family and are among the most important neurotransmitter receptors in the human body. Based on amino acid sequence homology and differences in intracellular signal transduction mechanisms, the eight mGluRs are classified into three groups: Group I includes mGluR1 and mGluR5, which mainly couple with Gq to activate phospholipase C, thereby promoting the hydrolysis of phosphatidylinositol 4,5-bisphosphate into inositol trisphosphate and diacylglycerol, resulting in elevated intracellular Ca²⁺concentration; Group II includes mGluR2 and mGluR3, which couple with Gi/o to inhibit the formation of cyclic adenosine monophosphate and voltage-sensitive Ca²⁺ channels, and to activate K⁺ channels; Group III includes mGluR4, mGluR6, mGluR7, and mGluR8, which also couple with Gi/o to inhibit the activity of adenylyl cyclase. To date, researchers have fused melanopsin and MW-opsin respectively with mGluR6 (ICL2, ICL3, CT) and expressed them in retinal bipolar cells, which has enabled partial vision restoration in blind rd1 mice. This strategy of treating retinal neurodegenerative diseases with chimeric proteins formed by fusing photoreceptive proteins and mGluRs is currently still at an early stage of development.

US20140171376A1 describes a chimeric GPCR protein suitable for coupling a light signal with the signal transduction cascade of mGluR6 in inner retinal ON bipolar cells. The chimeric GPCR protein comprises domains derived from at least two members of the G protein-coupled receptor (GPCR) family, characterized in that a first one of the at least two members contributes at least some of the domains that mediate light activation, namely a photoreceptive GPCR, and a second one of the at least two GPCR family members contributes at least some of the domains that enable coupling of light activation with the signal transduction cascade of mGluR6. Fusion of the endogenous photoreceptive protein melanopsin with mGluR6 has been shown to restore vision in blind rd1 mice.

US20230049217A1 describes a recombinant expression vector comprising a first inverted terminal repeat (ITR) polynucleotide sequence, a promoter polynucleotide sequence operatively linked to a polynucleotide sequence encoding a medium wavelength cone opsin (MW-opsin) transgene, a polyA polynucleotide sequence, and a second ITR polynucleotide sequence.

CN111417339A describes a method for restoring or enhancing visual function in an individual, the method comprising administering to the individual a nucleic acid comprising a nucleotide sequence encoding one or more of medium-wavelength cone opsin (MW-opsin), long-wavelength cone opsin (LW-opsin), and short-wavelength cone opsin (SW-opsin), wherein one or more of the MW-opsin, LW-opsin, and SW-opsin are expressed in retinal cells of the individual so as to restore or enhance visual function.

WO2021105509A1 describes chimeric opsin-mGluR6 proteins that exhibit one or more particularly advantageous properties and can be used in gene therapy for patients suffering from partial or complete vision loss caused by the lack or insufficiency of light-sensitive signal transduction activity provided by natural photoreceptors. Fusion of the endogenous photoreceptive protein melanopsin with mGluR6, or fusion of the endogenous photoreceptive protein MW-opsin with mGluR6, has been shown to restore vision in blind rd1 mice.

Gaub, Benjamin M et al. (Optogenetic Vision Restoration Using Rhodopsin for Enhanced Sensitivity, 2015,23(10),1562-1571, 2015, 23(10), 1562-1571) describes that endogenous light-sensing protein rhodopsin can be used to restore vision.

Therefore, efforts in the art have been directed to identifying GPCR chimeric opsins with low immunogenicity, high sensitivity, strong efficacy, and/or broad distribution, for the treatment of various retinal neurodegenerative diseases.

### Summary of the Present Invention

The inventors of the present invention surprisingly discover that by using other retinal neurons (retinal ganglion cells) to express GPCR chimeric opsins, blind mice can regain their vision, providing a new therapeutic strategy for restoring vision to blind patients through similar means in the clinic. Moreover, the GPCR chimeric opsin has low immune rejection, high sensitivity, strong efficacy and/or wide distribution. In addition, the use of the GPCR chimeric opsin (mGluR4 chimeric opsin) of the present invention may potentially achieve better effects in improving vision and/or treating various retinal neurodegenerative diseases than the mGluR6 chimeric opsin in the conventional technology. Furthermore, the GPCR chimeric opsin of the present invention (mGluR4 chimeric opsin) can be expressed in target cells different from the mGluR6 chimeric opsin in the conventional technology (The mGluR4 chimeric opsin of the present invention can be expressed in retinal ganglion cells while the mGluR6 chimeric opsin in the conventional technology is expressed in bipolar cells).

In one aspect, the present invention provides a GPCR chimeric opsin comprising a recombinant protein, wherein the recombinant opsin is formed by replacing the corresponding domain of the native opsin with at least one domain derived from mGluR4. In some embodiments, the native opsin is medium wavelength opsin (MW-opsin). In some embodiments, the at least one domain derived from mGluR 4 is selected from the group consisting of ICL2, ICL3, C-terminus, and combinations thereof. In some embodiments, the at least one domain derived from mGluR 4 is selected from the group consisting of ICL2, ICL3, and C-terminus. In some embodiments, the chimeric opsin comprises or consists of the amino acid sequence identified in SEQ ID NO: 3:

MAQQWSLQRLAGRHPQDSYEDSTQSSIFTYTNSNSTRGPFEGPNYHIAPRWVYH
LTSVWMIFVVIASVFTNGLVLAATMKFKKLRHPLNWILVNLAVADLAETVIASTI
SVVNQVYGYFVLGHPMCVLEGYTVSLCGITGLWSLAIISWERIYRIFEQGKRSVS
APRFISPASQVGIAFSWIWAAVWTAPPIFGWSRYWPHGLKTSCGPDVFSGSSYPG
VQSYMIVLMVTCCITPLSIIVLCYLQVWLKTRGVPETFNEAKMVVVMVLAFCFC
WGPYAFFACFAAANPGYPFHPLMAALPAFFAKSATIYNPVIYVFMNRQPEQNVP
KRKRSLKAVVTAATMSNKFTQKGNFRPNGEAKSELCENLEAPALATKQTYVTY
TNHAI (SEQ ID NO:3).

In some embodiments, the chimeric opsin further comprises other light-sensitive ion channel proteins, such as chr2, chrimsonR, MCO, etc., or their functional derivatives. In some embodiments, the recombinant opsin is formed by replacing the ICL2, ICL3 and C-terminus of the native opsin with ICL2, ICL3 and C-terminus derived from mGluR4. In some embodiments, the ICL2, ICL3 and C-terminus derived from mGluR4 respectively comprise the sequence IYRIFEQGKRSVSAPRFISPASQ (SEQ ID NO:5), KTRGVPETFNEAK (SEQ ID NO:6) and PEQNVPKRKRSLKAVVTAATMSNKFTQKGNFRPNGEAKSELCENLEAPALATK QTYVTYTNHAI (SEQ ID NO:7), or is formed by the sequence. In some embodiments, the GPCR chimeric opsin further comprises a signal peptide at the C-terminus. For example, a Golgi export signal sequence such as RSRFVKKDGHCNVQFINV (SEQ ID NO:8) and/or a rhodopsin trafficking sequence such as TETSQVAPA (SEQ ID NO:9). In some embodiments, the chimeric opsin comprises or consists of the amino acid sequence identified in SEQ ID NO: 1.

In another aspect, the present invention provides a nucleic acid molecule comprising a nucleotide sequence encoding the GPCR chimeric opsin described herein. In some embodiments, the nucleic acid molecule comprises or consists of the nucleotide sequence identified in SEQ ID NO: 4.

ATGGCTCAGCAATGGAGTTTGCAGAGACTGGCTGGTAGGCATCCTCAGGATA
GCTACGAGGACAGCACTCAGTCTAGTATTTTTACCTACACAAACTCCAACAG
CACAAGAGGCCCTTTCGAGGGCCCGAATTACCACATAGCGCCCAGGTGGGTG
TACCATCTTACTAGCGTTTGGATGATCTTTGTGGTGATCGCGTCCGTGTTCACT
AACGGGCTGGTGCTGGCTGCTACTATGAAGTTCAAAAAGCTGCGGCATCCAC
TGAACTGGATACTGGTTAATCTCGCCGTGGCCGACCTGGCTGAGACAGTTATC
GCTAGCACCATTTCTGTTGTGAATCAGGTCTACGGGTATTTTGTCCTCGGGCA
TCCAATGTGTGTGCTGGAAGGCTATACGGTAAGCCTTTGCGGAATCACCGGC
CTGTGGAGCCTCGCAATAATCTCCTGGGAACGAATTTACCGGATCTTCGAGC
AAGGGAAACGATCTGTGAGTGCCCCTAGGTTCATCAGTCCCGCCAGCCAGGT
TGGAATTGCCTTTAGCTGGATTTGGGCAGCTGTATGGACAGCACCACCAATTT
TTGGATGGAGCAGATATTGGCCCCACGGGCTCAAAACCAGCTGTGGCCCTGA
TGTGTTTTCTGGATCATCCTACCCTGGTGTTCAGAGTTATATGATTGTGTTGAT
GGTTACATGCTGCATCACACCGCTGTCAATCATCGTGCTTTGCTATCTCCAGG
TCTGGCTCAAGACTAGGGGGGTCCCTGAAACCTTTAATGAGGCCAAAATGGT
CGTCGTGATGGTGCTGGCTTTCTGTTTCTGCTGGGGGCCCTATGCTTTCTTTGC
ATGCTTCGCAGCCGCCAATCCTGGCTACCCATTTCATCCACTGATGGCTGCTT

TGCCAGCGTTTTTCGCGAAAAGTGCTACTATCTACAATCCCGTCATCTACGTG
TTTATGAACAGACAACCCGAACAGAACGTCCCAAAGAGAAAGAGGTCACTG
AAGGCTGTGGTTACCGCCGCCACTATGTCAAACAAGTTTACCCAGAAGGGTA
ATTTTCGCCCCAATGGAGAGGCAAAGTCCGAGCTTTGTGAGAATTTGGAGGC
GCCCGCCCTGGCTACCAAACAAACATATGTGACATACACCAATCACGCCATC
(SEQ ID NO:4).

In some embodiments, the nucleic acid molecule comprises or consists of the nucleotide sequence identified in SEQ ID NO: 2. In some embodiments, the nucleic acid molecule is an isolated nucleic acid molecule.

In another aspect, the present invention provides a vector comprising a nucleotide sequence encoding a GPCR chimeric opsin as described herein, or comprising a nucleic acid molecule as described herein. In some embodiments, the vector is an AAV vector. In some embodiments, the nucleotide sequence encoding a GPCR chimeric opsin as described herein is operatively linked to a bipolar cell-specific promoter (e.g., mGlu6) or a ganglion cell-specific promoter (e.g., SNCG), preferably a ganglion cell-specific promoter (e.g., SNCG).

In another aspect, the present invention provides a transgenic cell comprising the GPCR chimeric opsin as described herein, the nucleic acid molecule as described herein, or the vector as described herein. In some embodiments, the transgenic cell is a transgenic retinal ganglion cell. In some embodiments, the cell is a mammalian cell. In some embodiments, the cell is a human cell. In some embodiments, the cell is a non-human cell. In some embodiments, the cell is a retinal cell. In some embodiments, the cell is a bipolar cell or a ganglion cell. In some embodiments, the cell is in vitro. In some embodiments, the cell is in vivo.

In another aspect, the present invention provides the use of the GPCR chimeric opsin as described herein, the nucleic acid molecule as described herein, the vector as described herein, or the transgenic cell as described herein in the preparation of a medicament for improving vision of a subject in need thereof. In some embodiments, the subject is a mammal, such as a human. In some embodiments, the improvement comprises administering the GPCR chimeric opsin as described herein, the nucleic acid molecule as described herein, or the vector as described herein to a retinal ganglion cell. In some embodiments, the transgenic cell as described herein is a transgenic retinal ganglion cell.

In another aspect, the present invention provides the use of the GPCR chimeric protein described herein, the nucleic acid molecule described herein, the vector described herein, or the transgenic cell described herein in the preparation of a medicament for treating a retinal neurodegenerative disease in a subject in need thereof. In some embodiments, the retinal neurodegenerative disease is retinitis pigmentosa (RP) or age-related macular degeneration (AMD). In some embodiments, the subject is a mammal, such as a human. In some embodiments, the treatment comprises administering the GPCR chimeric opsin as described herein, the nucleic acid molecule as described herein, or the vector as described herein to a retinal ganglion cell. In some embodiments, the transgenic cell as described herein is a transgenic retinal ganglion cell.

In another aspect, the present invention provides a composition comprising the GPCR chimeric opsin as described herein, the nucleic acid molecule as described herein, the vector as described herein, or the transgenic cell as described herein.

In another aspect, the present invention provides a pharmaceutical composition comprising the GPCR chimeric opsin as described herein, the nucleic acid molecule as described herein, the vector as described herein, or the transgenic cell as described herein, and a pharmaceutically acceptable carrier.

In another aspect, the present invention provides a cell which is transduced with or comprises a viral vector as described herein. In one embodiment, the cell is a mammalian cell. In one embodiment, the cell is a human cell. In one embodiment, the cell is a non-human cell. In one embodiment, the cell is a retinal cell. In one embodiment, the cell is a bipolar cell or a ganglion cell. In one embodiment, the cell is in vitro. In one embodiment, the cell is in vivo.

In another aspect, the present invention provides a method for treating a retinal neurodegenerative disease, the method comprising administering a therapeutically effective amount of a GPCR chimeric opsin as described herein, a nucleic acid molecule as described herein, a vector as described herein, a transgenic cell as described herein, or a composition or pharmaceutical composition as described herein to the eye of a subject in need thereof. In some embodiments, the retinal neurodegenerative disease is retinitis pigmentosa (RP) or age-related macular degeneration (AMD). In some embodiments, the subject is a mammal, such as a human. In some embodiments, the method comprises administering the GPCR chimeric opsin as described herein, the nucleic acid molecule as described herein, or the vector as described herein to a retinal ganglion cell. In some embodiments, the transgenic cell as described herein is a transgenic retinal ganglion cell.

### Brief Description of the Drawings

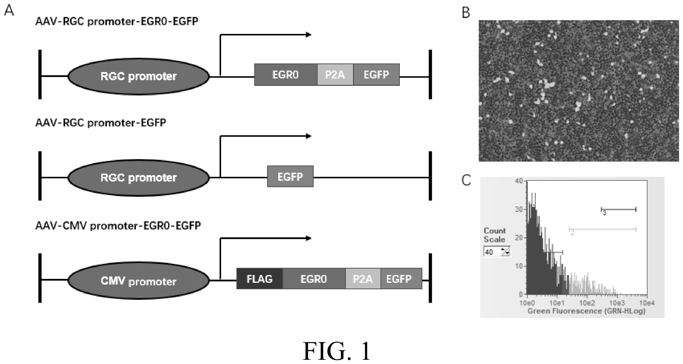

The present invention will be better understood with reference to the accompanying drawings
FIG. 1 illustrates the construction and in vitro verification of a AAV expression vector of GPCR chimeric opsin. A: schematic diagram of AAV expression vector of GPCR chimeric opsin. B: GPCR chimeric opsin EGRO C-terminus is co-expressed with green fluorescent protein EGFP through P2A and transfected into 293T cells in vitro, and imaging reveals EGFP expression, indirectly indicating that EGRO can be normally folded and expressed. C: Flow sorting and detection by using the FLAG tag, which reveals that EGRO can be normally and accurately positioned on the surface of the cell membrane.

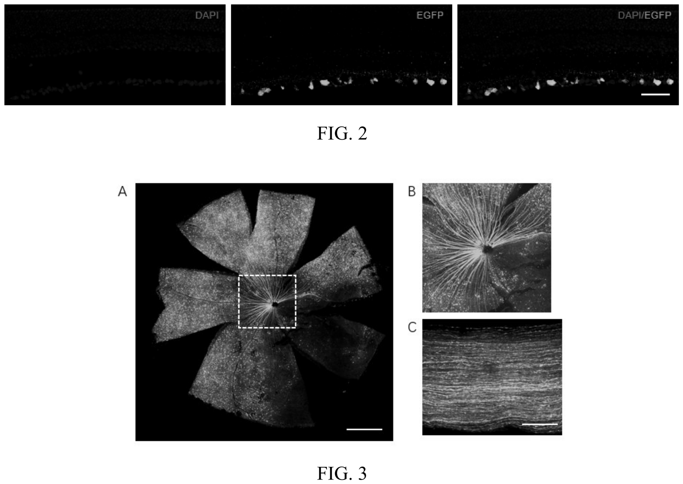

FIG. 2 illustrates that the RGC promoter is specifically expressed in retinal ganglion cell. Adult normal C57 mice (12 weeks old) are injected with AAV via intravitreal injection, and confocal imaging of retinal frozen sections after 2 weeks shows that EGFP is specifically expressed in retinal ganglion cells. Wherein the left panel shows a DAPI image (blue), the middle panel shows an EGFP image (green), and the right panel shows a DAPI and EGFP fused image (blue + green).
FIG. 3 illustrates the high-level expression of EGRO in retinal ganglion cells. C3H mice (12 weeks old) with photoreceptor degeneration-type blindness are injected with AAV via intravitreal injection, after 7 weeks, retinal flat mounts show that EGRO is highly expressed in retinal ganglion cells (A), whose axons converge in the optic nerve (C), and (B) is a local image of the optic papilla position.

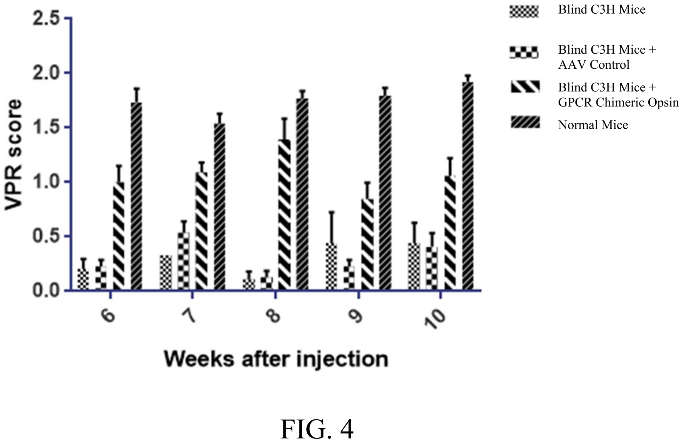

FIG. 4 illustrates the visual placement response score of C3H mice at different times after administration.

### Detailed Description of the Preferred Embodiment

The technical solutions in the embodiments of the present invention will be clearly and completely described below with reference to the accompanying drawings in the embodiments of the present invention. Obviously, the described embodiments are only some of the embodiments of the present invention, rather than all the embodiments. Based on the embodiments of the present invention, all other embodiments obtained by those of ordinary skill in the art without exerting creative efforts fall within the scope of protection of the present invention.

Retinal neurodegenerative diseases such as retinitis pigmentosa and age-related macular degeneration ultimately lead to the degeneration and apoptosis of retinal photoreceptor cells, resulting in irreversible damage to vision. In the present invention, through gene therapy, a GPCR chimeric opsin with light sensitive function is expressed in retinal ganglion cells by using adeno-associated virus (AAV) as a delivery vector, allowing these cells to retain their own physiological functions while also having the ability to sense exogenous light.

The GPCR chimeric opsin developed by the present invention is formed by replacing the ICL2, ICL3 and CT domains of MW-opsin with the ICL2, ICL3 and CT domains of mGluR4, respectively. MGluR4 is widely distributed in retinal ganglion cells. Therefore, without being bound by theory, if the GPCR chimeric opsin is specifically expressed in retinal ganglion cells and localized on the cell membrane surface, the light-sensitive portion of the MW-opsin is responsible for sensing exogenous light, the chimeric opsin is activated, and the mGluR4 portion utilizes the retinal ganglion cell's own endogenous downstream signaling pathway (GPCR-G protein pathway) to further integrate the information, which is ultimately transmitted to the brain's visual center through the optic nerve in the form of nerve impulses, i.e., visual restoration.

The present invention utilizes this treatment strategy, After expressing GPCR chimeric opsin in C3H adult mice with photoreceptor degeneration-type blindness (>7 weeks), the placement reaction shows that their behavior is partially restored. That is, after GPCR chimeric opsin is expressed in blind mice, the entire eye-brain visual pathway can be repaired.

In the present invention, unless otherwise specified, the use of the singular includes the plural, the word "a/one kind" may mean "at least one/ one kind", "or" is used to mean "and/or". In addition, the open-ended expressions "include" and "comprise" are interpreted as also including structural components or method steps that are not mentioned, but it should be noted that the open-ended expressions also cover situations consisting only of the described components and method steps (i.e., they cover situations with the closed-ended expression "consisting of...").

In the present description, the term "about," when used in conjunction with a percentage or other quantity, means the percentage or quantity plus or minus 10%. For example, "about 80%" includes 80% plus or minus 8%.

In the present application, all documents cited, including but not limited to patents, patent applications, articles, books, and treaties, are hereby expressly incorporated herein by reference into this application for any purpose. If the definition of a term in one or more of the incorporated literature and similar materials conflicts with the definition of that term in the present application, the definition in the present application shall prevail.

In the present description, the terms "protein," " polypeptide, "and" peptide " are interchangeable unless otherwise indicated.

As used in the present invention, unless otherwise specified, the terms "treatment" and "therapy" refer to an action that occurs when a subject is suffering from a disease, such as a retinal neurodegenerative disease, thereby reducing the severity of one or more symptoms or the effect of the disease. As used in the present invention, unless otherwise specified, the term "prevent" refers to an action that occurs before a subject begins to have a disease, such as a retinal neurodegenerative disease, which delays the onset of the disease, and/or inhibits or reduces the severity of the disease. It should be understood that the treatment may be a prophylactic treatment or a treatment performed after a disease or condition is diagnosed. The treatment of the present invention may reduce or eliminate symptoms or characteristics of a disorder, disease or condition, or may also eliminate the disorder, disease, or condition itself. It should be understood that the therapeutic methods of the present invention can slow or eliminate the progression of a disease, disorder, or condition, and in some cases can lead to regression of the disease, disorder, or condition. In some embodiments of the present invention, the GPCR chimeric opsins of the present invention can be expressed in a population of cells and used to treat retinal neurodegenerative diseases.

As used in the present invention, unless otherwise specified, a "therapeutically effective amount" of a compound is an amount sufficient to provide any therapeutic benefit in the treatment of a disease, or an amount sufficient to delay or alleviate one or more symptoms associated with the disease. A therapeutically effective amount of a compound refers to an amount of the compound, used alone or in combination with one or more other therapies and therapeutic agents, that provides any therapeutic benefit in the treatment of a disease. The term "therapeutically effective amount" can include an amount that alleviates retinal neurodegenerative disease, improves or reduces visual impairment, improves overall treatment, or enhances the efficacy of another therapeutic agent.

As used in the present invention, a "patient" or "subject" includes mammals suffering from or susceptible to suffering from a disease described herein, such as humans and non-human mammals. Non-limiting examples of non-human mammals include rodents, mice, rats, non-human primates, companion animals (such as dogs and cats), and livestock (such as sheep, cattle, horses, etc.).

As used in the present invention, "vision" or "eyesight" are used interchangeably and are defined as the ability of an organism to effectively detect light as a stimulus. "Vision" or "eyesight" is intended to encompass the following: (i) light detection or perception, i.e., the ability to discern the presence or absence of light; (ii) light projection, i.e., the ability to discern the origin of a light stimulus; (iii) resolution, i.e., the ability to detect varying brightness levels (i.e., contrast) within a grid or lettered target; and (iv) recognition, i.e., the ability to identify the shape of a visual object by reference to varying contrast within the target. Thus, "vision" or "eyesight" includes the ability to simply detect the presence of light, preferably visible light, more preferably light having a wavelength between about 365 nm and about 700 nm, between about 480 nm and about 590 nm, and in some embodiments, peak activation can occur upon exposure to light of about 530 nm.

As used in the present invention, "functional derivative" include "mutant", "variant" and "fragment", whether these terms are used in conjunction or interchangeably. Although conservative substitutions of, for example, 2, 3, 4, or 5 residues are also consistent with the present invention, preferred variants are single amino acid conservative substitution variants. In some embodiments, the functional derivative is at least 70% homologous to the full-length amino acid sequence of the parent polypeptide, preferably at least 75% homologous, more preferably at least 80% homologous, more preferably at least 85% homologous, more preferably at least 90% homologous, more preferably at least 95% homologous, more preferably at least 99% homologous, and more preferably 100% homologous. The percentage of homology is determined based on the length of the relevant amino acid sequences. Thus, if the polypeptide according to the present invention is comprised within a larger polypeptide, the percentage of homology is determined only with respect to the portion of the polypeptide corresponding to the polypeptide according to the present invention and not with respect to the entire larger polypeptide. "Homology percentage " with respect to polypeptide sequences refers to the percentage of identical amino acids between at least two polypeptide sequences aligned using the Base Local Alignment Search Tool (BLAST) engine. The BLAST engine is provided to the public by the National Center for Biotechnology Information (NCBI) in Bethesda, Maryland. According to a specific embodiment, a functional derivative is a polypeptide comprising an amino acid sequence that is at least 70% homologous to the full-length sequence of the parent polypeptide, wherein it differs from the parent polypeptide only by substitutions at one or more positions. Such substitutions are preferably "conservative substitutions". In addition, the functional derivative has at least 70% identity with the full-length amino acid sequence of the parent polypeptide,, preferably at least 75% identity, more preferably at least 80% identity, more preferably at least 85% identity, more preferably at least 90% identity, more preferably at least 95% identity, more preferably at least 99% identity, more preferably 100% identity. Methods for determining sequence identity or homology are known in the art.

Functional derivatives may comprise amino acid residues that are not present in the corresponding native protein or are missing relative to the corresponding native protein. Functional derivatives may also be truncated "fragments" compared to the corresponding native protein, i.e., only a portion of the full-length protein. Functional derivatives also include peptides having at least one D-amino acid.

As used in the present invention, the term "conservative substitution" generally refers to an amino acid substitution that maintains the structural and functional properties of a protein or polypeptide. Such functionally equivalent (conservatively substituted) peptide amino acid sequences include, but are not limited to, additions or substitutions of amino acid residues within the amino acid sequence encoded by the nucleotide sequence that result in silent changes, thereby producing a functionally equivalent gene product. Conservative substitution of amino acid can be made based on similarity in polarity, charge, solubility, hydrophobicity, hydrophilicity, and/or amphipathic properties of the relevant residues. For example: non-polar (hydrophobic) amino acids include alanine, leucine, isoleucine, valine, proline, phenylalanine, tryptophan, and methionine; polar neutral amino acids include glycine, serine, threonine, cysteine, tyrosine, asparagine, and glutamine; positively charged (basic) amino acids include arginine, lysine, and histidine; and negatively charged (acidic) amino acids include aspartic acid and glutamic acid.

Conservative substitution of amino acid may also be performed based on the hydrophilic index of amino acid. According to the hydrophobicity and charge characteristics of each amino acid, a hydrophilic index is assigned. They are: isoleucine (+4.5); valine (+4.2); leucine (+3.8); phenylalanine (+2.8); cysteine/ cystine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamate (-3.5); glutamine (-3.5); aspartic acid (-3.5); asparagine (-3.5); lysine (-3.9); and arginine (-4.5). The use of the hydropathic amino acid index in conferring interactive biological function on proteins is known in the art (Kyte and Doolittle, J. Mol. Biol., 157:105-132, 1982). It is known that, in certain circumstances, certain amino acids can be substituted for other amino acids having a similar hydropathic index or score and still retain similar biological activity. When making changes based on similar hydropathic indices, some embodiments include substitutions of amino acids within the range of +-2, other embodiments include substitutions within the range of +-1, and still other embodiments include substitutions within the range of +-0.5.

Conservative substitution of amino acid may also be made on the basis of hydrophilicity, particularly in cases where the resulting biologically functional protein or peptide is intended for immunological applications. In certain embodiments, the maximum local average hydrophilicity of a protein (determined by the hydrophilicity of its adjacent amino acids) correlates with its immunogenicity and antigenicity, i.e., with the biological properties of the protein. The hydropathicity values of these amino acid residues are assigned as follows: arginine (+3.0); lysine (+3.0); aspartic acid (+3.0 +-1); glutamic acid (+3.0 +-1); serine (+0.3); asparagine (+0.2); glutamine (+0.2); glycine (0); threonine (-0.4); proline (-0.5 +-1); alanine (-0.5); histidine (-0.5); cysteine (-1.0); methionine (-1.3); valine (-1.5); leucine (-1.8); isoleucine (-1.8); tyrosine (-2.3); phenylalanine (-2.5) and tryptophan (-3.4). When making changes based on similar hydropathicity values, some embodiments include substitutions of amino acids within a hydropathicity range of +-2, other embodiments include substitutions within a hydropathicity range of +-1, and still other embodiments include substitutions within a hydropathicity range of +-0.5.

Changes in amino acid are achieved by altering the codons in the corresponding nucleic acid sequence. It is known that such polypeptides can be obtained by substituting certain amino acids with other amino acids within the polypeptide structure in order to modify or improve biological activity. For example, by substituting alternative amino acids, small conformational changes can be imparted to a polypeptide that result in increased activity. Alternatively, amino acid substitutions in certain polypeptides can be used to provide residues that can then be linked to other molecules to provide peptide-molecule conjugates that retain sufficient properties of the starting polypeptide to be used for other purposes.

In some embodiments, the GPCR chimeric opsins of the present invention include functional derivatives thereof. In some embodiments, the mGluR4 or native opsins of the present invention include functional derivatives thereof.

Metabotropic glutamate receptor 4 (mGluR4) belongs to the Group III metabotropic glutamate receptor (mGluR) subfamily and is prominently expressed at presynaptic sites in the central nervous system (Benitez et al., 2000; Bradley et al., 1996; Bradley et al., 1999; Mateos et al., 1998; Phillips et al., 1997), where it can act as both an autoreceptor and a heteroreceptor to regulate the release of GABA and glutamate. Metabotropic glutamate receptor 4 (mGluR4) has also been shown to be expressed at low levels in some postsynaptic sites (Benitez et al., 2000). Numerous reports indicate that metabotropic glutamate receptor 4 (mGluR4) is most commonly expressed in brain regions, particularly in neurons known to be critical for basal ganglia function (Bradley et al., 1999; Corti et al., 2002; Kuramoto et al., 2007; Marino et al., 2003a), in neurons known to play a key role in learning and memory (Bradley et al., 1996), in neurons known to be play a key role in vision (Akazawa et al., 1994; Koulen et al., 1996; Quraishi et al., 2007), in neurons known to play an important role in cerebellar function (Makoff et al., 1996), in neurons known to play an important role in the regulation of diet and hypothalamic hormones (Flor et al., 1995), in neurons known to play an important role in sleep and wakefulness (Noriega et al., 2007), and expressed in other neurons.

In the present description, the term "domain" refers to the intracellular and extracellular loops, N- and C-termini, and transmembrane regions of GPCR protein family members, such as mGluR4 or opsin. The term "at least one domain derived from mGluR4" includes any domain, such as the transmembrane domain, the three extracellular loops (ECL1, ECL2, ECL3), the three intracellular loops (ICL1, ICL2, ICL3), the N-terminal domain (NT), the C-terminal domain (CT), or their physiologically relevant counterparts, wherein the physiologically relevant counterparts have an amino acid sequence identical to or similar to the sequence of such domains in the physiological counterparts of mGluR4. In general, similar amino acid sequences or similar domains exhibit at least 60% homology, preferably at least 80% homology, and most preferably at least 90% homology. Similar domains also specifically include domains containing conservative substitution of amino acid. In some embodiments of the present invention, the at least one domain derived from mGluR4 constitutes a binding site for a downstream G protein required for mGluR function. In some embodiments of the present invention, the at least one domain derived from mGluR4 comprises an intracellular domain of mGluR4 capable of coupling light activation to the signal transduction cascade of mGluR4.

Opsin is a membrane protein with a molecular weight of about 30-50 kDa. It consists of an extracellular amino terminus, seven transmembrane regions (a transmembrane domain, three extracellular loops (ECL1, ECL2, and ECL3), three intracellular loops (ICL1, ICL2, and ICL3), and an intracellular carboxyl terminus. It belongs to the G protein-coupled receptor (GPCR) superfamily. Opsins are widely distributed in animals and microorganisms. Non-animal opsins from archaea, bacteria, and fungi share similar three-dimensional structures with animal opsins, but their amino acid sequences differ significantly. Studies have confirmed that animal opsins are diverse and widely distributed, and they have non-visual functions such as visual perception, regulating circadian rhythms, and participating in the pupillary light reflex. Generally speaking, vertebrate retinas contain two types of photoreceptor cells: rod cells and cone cells. The opsin in rod cell is rhodopsin (Rh), which is associated with scotopic vision; the cone cell contains cone opsins, which are associated with photopic vision. Based on their absorption spectrum, cone opsins can be further divided into longwave-sensitive opsin (LW), mediumwave-sensitive opsin (MW), and shortwave-sensitive opsin (SW). The latter can be further divided into SW1 and SW2. Most vertebrate retinas contain the four cone opsins described above, but SW2 and MW opsins are not found in most mammals. Vertebrate visual opsins include rhodopsin and cone opsins. In some embodiments of the present invention, the opsin is a visual opsin from a vertebrate, such as a mammal, for example, a human. In some embodiments of the present invention, the opsin is a cone opsin, particularly a MW-opsin.

According to the present invention, the phrase "replacing the corresponding domain of a native opsin with at least one domain derived from mGluR4" means that one or more domains of opsin are changed to the corresponding domain of mGluR4 by techniques well known in the art, such as recombinant techniques. For example, in some embodiments, one or more of the intracellular loops, extracellular loops, N-terminus, C-terminus, and transmembrane region of opsin can be replaced with one or more of the intracellular loops, extracellular loops, N-terminus, C-terminus, and transmembrane region, respectively, derived from mGluR4. For example, in some embodiments, the intracellular loops of opsin can be replaced with those derived from mGluR4, and the C-terminus of opsin can be replaced with the C-terminus derived from mGluR4. For example, in some embodiments, ICL2, ICL3, and the C-terminus of opsin can be replaced with ICL2, ICL3, and the C-terminus derived from mGluR4, respectively. In some embodiments, the act of replacing couples light activation to the signaling cascade of mGluR4.

According to the present invention, the chimeric opsin can be expressed in specific cell, tissue, and/or organism and used to control the response of cell to light pulse of appropriate wavelength in vivo, ex vivo, and in vitro. According to techniques known in the art, GPCR chimeric opsin are constructed by replacing at least one domain derived from mGluR4 with the corresponding domain of opsin together with light activation to the signal transduction cascade of mGluR4. The identification of the desired domain and determination of suitable cleavage and ligation site at the N- and C-termini of any particular domain are primarily based on: 1) the alignment of the gene sequence/ conservative residues; and 2) processing computer modeling of the secondary and tertiary structures of photoreceptor opsins and mGluR4 by using standard software available in the art. This approach has inherent variability in the exact definition of the length of each domain, and such variability is encompassed within the scope of the present invention when referring to domains. Furthermore, at each fusion site between domains, there are generally multiple possibilities for splicing the domains together to produce a functional protein. It is also apparent that deletions, conservative substitution of amino acid and nucleotide substitution of portion of the amino acid sequence not required for function (such as exchanging hydrophobic amino acids for hydrophobic amino acids or hydrophilic amino acids for hydrophilic amino acids) are also within the scope of the present invention. Thus, a wide range of sequence variants fall within the scope of the present invention, provided they produce functional GPCR chimeric opsins (particularly in the region of the fusion site between the adjacent domains of the GPCR and opsin). In some embodiments, the GPCR chimeric opsin of the present invention may further comprise a signal peptide at the C-terminus to promote its expression and/or trafficking. For example, sequence of a Golgi export signal such as RSRFVKKDGHCNVQFINV (SEQ ID NO: 8) and/or, for example, a rhodopsin trafficking sequence such as TETSQVAPA (SEQ ID NO: 9).

According to the present invention, the term "ion channel" refers to a transmembrane polypeptide that forms a pore that, when activated, opens, allowing ion conduction through the pore across the membrane. According to the present invention, the light-sensitive ion channel protein includes chr2, chrimsonR, MCO or a functional derivative thereof.

According to the present invention, the chimeric opsin or light-sensitive ion channel protein is strongly activated by exposure to visible light, preferably light having a wavelength between about 365 nm and about 700 nm, or light having a wavelength between about 480 nm and about 590 nm, and in some embodiments, peak activation occurs when exposed to light with a wavelength of about 530 nm.

Although the peptide amino acid sequences of the present invention can be chemically synthesized, a large polypeptide sequence can preferably be produced by recombinant DNA technology using techniques well known in the art for expressing nucleic acids containing nucleotide sequences encoding the desired peptide. Such methods can be used to construct expression vectors containing the nucleotide sequence encoding the peptide and appropriate transcriptional and translational control signals. These methods include, for example, in vitro recombinant DNA techniques, synthetic techniques, and in vivo gene recombination, which are well known in the art.

Some specific embodiments provide an isolated nucleic acid comprising a nucleotide sequence encoding a chimeric opsin protein or a functional derivative thereof according to the present invention. In some specific embodiments, the nucleic acid comprises or consists of the nucleotide sequence identified in SEQ ID NO: 2 or 4. In other specific embodiments, the present invention provides an isolated nucleic acid comprising a nucleotide sequence encoding a chimeric opsin protein or a functional derivative thereof according to the present invention, wherein the nucleotide sequence has at least 70% identity with SEQ ID NO: 2 or 4, preferably at least 75% identity, more preferably at least 80% identity, more preferably at least 85% identity, more preferably at least 90% identity, more preferably at least 95% identity, more preferably at least 99% identity, and more preferably 100% identity.

According to the present invention, the nucleic acid may include additional sequences including, but not limited to, one or more signal sequences (e.g., enhancers, polyadenylation signals, additional restriction enzyme sites, multiple cloning sites) and/or promoter sequences, or other encoding segments, or combinations thereof. The promoter may be a general or cell-specific promoter, inducible or constitutive. One example of a cell-specific promoter is the bipolar cell-specific mGluR6 promoter or the ganglion cell-specific SNCG promoter, preferably the ganglion cell-specific SNCG promoter. Some specific embodiments are any of the disclosed methods, wherein the promoter is a constitutive promoter. Some embodiments are any of the methods disclosed, wherein the constitutive promoter includes, but is not limited to, a CMV promoter or a CAG promoter (composed of the cytomegalovirus (CMV) early enhancer and the chicken β-actin promoter). The promoter, vector, enhancer, and polyadenylation site are routinely selected by those skilled in the art. These elements are fully described in the literature and are commercially available.

According to the present invention, the term "vector" refers to a nucleic acid molecule capable of transporting another nucleic acid between different genetic environments, to which the nucleic acid molecule is operatively linked.

A nucleic acid is "operatively linked" when it is placed into a functional relationship with another nucleic acid sequence. In general, "operatively linked" means that the linked DNA sequence is continuous. However, the enhancer need not be continuous. Linking is achieved by ligation at conventional restriction sites. If such sites do not exist, synthetic oligonucleotide adapters or linkers are used according to conventional practice.

The term "vector" also refers to a virus or organism capable of transporting a nucleic acid molecule. One type of vector is an episome, a nucleic acid molecule capable of extrachromosomal replication. Some useful vectors are those capable of autonomous replication and/or expression of nucleic acids to which they are linked. Vectors capable of directing the expression of genes to which they are operatively linked are referred to herein as "expression vectors." Expression vectors and methods of using them are well known in the art. The present invention provides non-limiting examples of suitable expression vectors and methods of using them. According to a preferred embodiment, the vector is suitable for gene therapy, in particular for viral-mediated gene transfer. Viral vectors suitable for gene therapy include retroviruses, adenoviruses, adeno-associated viruses (AAV), lentiviruses, poxviruses (e.g., MVA), alphaviruses, herpes viruses, and the like. Examples of AAV can be found in Davidson et al., PNAS (2000) 97: 3428-3432. AAV and lentiviruses can confer persistent expression, while adenoviruses can provide transient expression.

However, gene therapy further includes non-viral approaches, such as the use of naked DNA or nucleic acids bound to liposomes. Vectors suitable for use in some methods according to the present invention can genetically insert chimeric opsins into dividing and non-dividing cells, and can insert chimeric opsins into cells in vivo, in vitro, or ex vivo.

In some preferred embodiments, the nucleic acid expression vector comprising the gene of the chimeric opsin protein according to the present invention is selected from an AAV viral vector. The term "serotype" refers to an AAV that is identified by a defined antiserum and distinguished from other AAVs based on capsid protein reactivity with the defined antiserum. For example, there are many known serotypes of primate AAV (such as AAV1, AAV2, AAV3, AAV4, AAV5, AAV6, AAV7, AAV8, AAV9, AAV10, AAV-Rh74, and AAV-Rh10, as well as modified capsids of these serotypes). "AAV virus" or "AAV viral particle" refers to a viral particle composed of at least one AAV capsid protein (preferably all capsid proteins of wild-type AAV) and a polynucleotide encapsidated by the capsid. According to a preferred embodiment of the present invention the AAV viral vector used is serotype AAV2, AAV9 or AAV2/7m8, more preferably serotype AAV2/7m8.

Some specific embodiments include a method of treating or preventing retinal neurodegenerative diseases, the method comprising: (a) delivering to a target cell a nucleic acid expression vector encoding a chimeric opsin or a functional derivative thereof according to the present invention, wherein the chimeric opsin or a functional derivative thereof is capable of being expressed in the target cell, and the vector comprises an open reading frame encoding the chimeric opsin or a functional derivative thereof according to the present invention, operatively linked to a promoter sequence and optionally operatively linked to a transcriptional regulatory sequence; (b) expressing the vector in the target cell, wherein the expressed chimeric opsin or functional derivative thereof activates the target cell upon exposure to light.

According to the present invention, chimeric opsin has been found to be suitable for expression and use in a mammalian cell under normal cellular environmental conditions and ionic concentrations, without the need for any chemical supplementation. According to the present invention, chimeric opsin has been found to be activated by light wavelengths ranging from 365 nm to 700 nm, with preferred activation wavelengths between 480 nm and 590 nm, and a peak activation wavelength at 530 nm.

An effective amount of a chimeric opsin protein, a functional derivative thereof, or a nucleic acid expression vector thereof is an amount that elevates the level of the chimeric opsin protein in a cell, tissue, or subject to a level that is beneficial to the subject. The effective amount can also be determined by evaluating the physiological impact on the administration of the cell or subject, such as a decrease in symptoms following administration. Other detections are known to those of ordinary skill in the art and can be used to determine the response level of the treatment. The amount of treatment can be varied, for example, by increasing or decreasing the amount of chimeric opsin or nucleic acid expression vector administered, changing the therapeutic composition containing the chimeric opsin or nucleic acid expression vector administered, changing the route of administration, changing the timing of administration, and the like. The effective amount will vary depending on the specific condition being treated, the age and physical condition of the subject being treated, the severity of the condition, the duration of treatment, the nature of concurrent therapy (if any), the specific route of administration, and similar factors within the knowledge and expertise of the physician. These factors are well known to those skilled in the art and can be addressed through routine experimentation. One of ordinary skill in the art will appreciate that the subject may adhere to the use of lower dose or tolerated dose due to medical reason, psychological reason, or virtually any other reason.

According to the present invention, chimeric opsin or functional derivatives thereof, or nucleic acid expression vector thereof, can be administered using methods known in the art. In certain embodiments, a nucleic acid encoding a chimeric opsin protein or a functional derivative thereof according to the present invention is administered to a subject. In certain embodiments, the chimeric opsin protein or its functional derivative thereof according is administered to a subject. The route of administration and dosage can be individually tailored by the physician, particularly if any complications arise. The absolute amount administered will depend on various factors, including the material selected for administration, whether single or multiple doses are administered, and the personal parameters of the subject, including age, weight, physical condition, and disease stage. These factors are well known to those skilled in the art and can be determined with only routine experimentation.

Pharmaceutical compositions comprising the chimeric opsin or its functional derivative or nucleic acid expression vector according to the present invention can be administered alone and/or in combination with other drug therapies or other treatment regimens administered to a subject. Various modes of administration are known to those of ordinary skill in the art. Suitable modes of administration may be topical, intravenous, oral, intracavitary, intrathecal, intrasynovial, buccal, sublingual, intranasal, transdermal, subvitreal, subretinal, subcutaneous, intramuscular and intradermal. The present invention is not limited by the particular mode of administration disclosed herein

The disclosure of the present invention, in certain aspects, comprises preparing a nucleic acid comprising a nucleotide sequence; expressing a polypeptide encoding the prepared nucleic acid and nucleotide sequence in a cell and/or a membrane; illuminating the cell and/or membrane with appropriate light and verifying a rapid depolarization of the cell and/or a change in transmembrane conductivity in response to the light, as well as a rapid recovery from the depolarization after the light fades. The ability to controllably alter transmembrane voltage and cell depolarization via light has been verified. The present invention makes it possible to make use of light to control cellular functions in vivo, ex vivo, and in vitro, and the chimeric opsin according to the present invention have a wide range of applications in drug screening, therapeutics, and research, some of which have been described in the present invention.

### Embodiments

The following embodiments provide further details about various specific embodiments. Those skilled in the art should understand that the technologies disclosed in the following embodiments represent the technologies and/or compositions that the inventor have discovered and can function well. However, according to the present invention, those skilled in the art will appreciate that many modifications may be made to the disclosed embodiments without departing from the spirit and scope of the present invention, and that similar or approximate results may be obtained from these modifications. These embodiments are for illustrative purposes only and are not intended to limit the scope of the present invention.

Unless otherwise specified, the materials used in the embodiments herein are commercially available, and the various specific experimental methods for conducting experiments are all conventional experimental methods in the art or steps and conditions suggested by the manufacturer, and can be routinely determined by those skilled in the art as needed. Certain materials and methods are described in detail below.

### Embodiment 1: Construction of GPCR Chimeric Opsin AAV Expression Vector and in vitro Cell Verification

In the full-length human GPCR chimeric opsin gene sequence (SEQ ID NO: 2) containing an expression-promoting signal peptide (Golgi export signal + rhodopsin trafficking sequence) at the C-terminus, which is synthesized at Nanjing GenScript Biotechnology Co., Ltd, through the traditional gene cloning method of enzyme ligation cloning, clone the above encoding gene into the expression vector driven by the retinal ganglion cell-specific promoter RGC promoter (SNCG), and simultaneously link the C-terminus of the gene to a reporter gene EGFP through P2A by means of Gibson assembly, a seamless cloning technique, to form AAV-RGC promoter-EGR 0-EGFP plasmid. The AAV is packaged in the later stage for in vivo efficacy verification experiments, and construct a control group with viral plasmid AAV-RGC promoter-EGFP plasmid. At the same time, a Flag label (HA label) is added at the N-terminus of the gene, the C-terminal is connected to the reporter gene EGFP through P2A and cloned into a broad-spectrum promoter CMV plasmid to form a CMV promoter-Flag-EGR 0-EGFP plasmid, which is used in the later stage for in vitro verification experiments. The plasmid is transfected into HEK293T cells in vitro, and the imaging reveal that the reporter gene EGFP can be expressed normally (FIG. 1b), while the flow cytometric analysis using Flag tag-specific antibodies reveals that Flag tag signals can be detected on the membranes of HEK293 cells transfected with the plasmid (FIG. 1c). Since EGRO is fused with the Flag tag and expressed, and the Flag tag is located outside the cell membrane, this proves that EGRO can be expressed normally and located on the cell membrane surface, which provides a structural basis for the treatment of retinal neurodegenerative diseases.

### Embodiment 2: AAV Administration in Mice

In this study, the AAV2/7m8, which is produced by Heyuan Biotechnology (Shanghai) Co., Ltd., and normal wild-type C57 mice and C3H mice with photoreceptor cell degeneration (12 weeks old, male) are selected and used for subvitreal injection. The dosage of administration is 1.5×10⁹ VG/ eye (1.5ul, 1×10¹² VG/ml).

### 1) RGC promoter specifically labels retinal ganglion cells

Confocal imaging of retinal frozen sections of normal wild-type C57 mice at 2 weeks after injection of AAV-RGC promoter-EGFP reveals that EGFP is specifically expressed in retinal ganglion cells (FIG. 2).

### 2) High-level expression of GPCR chimeric opsin in the retina of blind C3H mice after administration

Retinal flat mounts and optic nerve imaging of C3H mice 7 weeks after administration reveals that EGRO is highly expressed in retinal ganglion cells (FIG. 3a), and the axons converge in the optic nerve (FIG. 3c).

### 3) The behavior of blind C3H mice is partially improved after drug administration

Seven weeks after drug administration, visual placing reaction of C3H mice are tested, that is, the mouse's tail is lifted 15 cm above a metal cage lid and slowly approached towards the lid, bringing the mouse's head about 2-4 cm from the lid (1-2 seconds). Normal mice in this state will raise their heads and reach for the cage lid with their forelimbs, a reaction known as the placing response. However, visually impaired mice do not exhibit similar response because they are unable to see the cage lid. We score the placing response of C3H mice after drug administration, with a score of 0 indicating no placing response at all; 1 indicating a weak placing response; and 2 indicating a significant placing response. It is found that the placing reaction of blind C3H mice expressing GPCR chimeric opsin is significantly better than that of the control group, indicating that GPCR chimeric opsin can partially restore the behavior of blind mice, that is, the chimeric opsin can be used to treat retinal neurodegenerative diseases.

The sequence information of the GPCR chimeric opsin (EGRO) containing a signal peptide (Golgi export signal + rhodopsin trafficking sequence) at the C-terminus to promote expression is shown below:
Amino acid sequence: 
MAQQWSLQRLAGRHPQDSYEDSTQSSIFTYTNSNSTRGPFEGPNYHIAP
RWVYHLTSVWMIFVVIASVFTNGLVLAATMKFKKLRHPLNWILVNLAVADLAE
TVIASTISVVNQVYGYFVLGHPMCVLEGYTVSLCGITGLWSLAIISWERIYRIFEQ
GKRSVSAPRFISPASQVGIAFSWIWAAVWTAPPIFGWSRYWPHGLKTSCGPDVFS
GSSYPGVQSYMIVLMVTCCITPLSIIVLCYLQVWLKTRGVPETFNEAKMVVVMV
LAFCFCWGPYAFFACFAAANPGYPFHPLMAALPAFFAKSATIYNPVIYVFMNRQ
PEQNVPKRKRSLKAVVTAATMSNKFTQKGNFRPNGEAKSELCENLEAPALATK
QTYVTYTNHAIRSRFVKKDGHCNVQFINVTETSQVAPA
 (SEQ ID NO: 1, the underlined amino acid residues are the Golgi export signal sequence, while the bold and double-underlined ones are the rhodopsin transport sequence.)
Nucleotide sequence: 
ATGGCTCAGCAATGGAGTTTGCAGAGACTGGCTGGTAGGCATCCTCA
GGATAGCTACGAGGACAGCACTCAGTCTAGTATTTTTACCTACACAAACTCC
AACAGCACAAGAGGCCCTTTCGAGGGCCCGAATTACCACATAGCGCCCAGGT
GGGTGTACCATCTTACTAGCGTTTGGATGATCTTTGTGGTGATCGCGTCCGTG
TTCACTAACGGGCTGGTGCTGGCTGCTACTATGAAGTTCAAAAAGCTGCGGC
ATCCACTGAACTGGATACTGGTTAATCTCGCCGTGGCCGACCTGGCTGAGAC
 
AGTTATCGCTAGCACCATTTCTGTTGTGAATCAGGTCTACGGGTATTTTGTCC
TCGGGCATCCAATGTGTGTGCTGGAAGGCTATACGGTAAGCCTTTGCGGAAT
CACCGGCCTGTGGAGCCTCGCAATAATCTCCTGGGAACGAATTTACCGGATC
TTCGAGCAAGGGAAACGATCTGTGAGTGCCCCTAGGTTCATCAGTCCCGCCA
GCCAGGTTGGAATTGCCTTTAGCTGGATTTGGGCAGCTGTATGGACAGCACC
ACCAATTTTTGGATGGAGCAGATATTGGCCCCACGGGCTCAAAACCAGCTGT
GGCCCTGATGTGTTTTCTGGATCATCCTACCCTGGTGTTCAGAGTTATATGAT
TGTGTTGATGGTTACATGCTGCATCACACCGCTGTCAATCATCGTGCTTTGCT
ATCTCCAGGTCTGGCTCAAGACTAGGGGGGTCCCTGAAACCTTTAATGAGGC
CAAAATGGTCGTCGTGATGGTGCTGGCTTTCTGTTTCTGCTGGGGGCCCTATG
CTTTCTTTGCATGCTTCGCAGCCGCCAATCCTGGCTACCCATTTCATCCACTG
ATGGCTGCTTTGCCAGCGTTTTTCGCGAAAAGTGCTACTATCTACAATCCCGT
CATCTACGTGTTTATGAACAGACAACCCGAACAGAACGTCCCAAAGAGAAAG
AGGTCACTGAAGGCTGTGGTTACCGCCGCCACTATGTCAAACAAGTTTACCC
AGAAGGGTAATTTTCGCCCCAATGGAGAGGCAAAGTCCGAGCTTTGTGAGAA
TTTGGAGGCGCCCGCCCTGGCTACCAAACAAACATATGTGACATACACCAAT
CACGCCATCAGAAGCCGCTTTGTGAAGAAAGATGGTCATTGCAATGTGCAGT
TTATTAATGTGACCGAGACCTCACAGGTTGCCCCAGCC
 (SEQ ID NO: 2, the underlined nucleotide sequence encodes the Golgi export signal, while the bold and double-underlined nucleotide sequence encodes the rhodopsin trafficking sequence.)

## Claims

1. A GPCR chimeric opsin, **characterized in that**: comprising a recombinant opsin in which a domain of a native opsin is replaced with at least one domain derived from mGluR4.

2. The GPCR chimeric opsin, as recited in claim 1, **characterized in that**, the native opsin is a medium-wavelength opsin.

3. The GPCR chimeric opsin, as recited in claim 1 or claim 2, **characterized in that**, the at least one domain derived from mGluR4 is chosen from ICL2, ICL3, the C-terminus, or any combination thereof, preferably including ICL2, ICL3, and the C-terminus.

4. The GPCR chimeric opsin, as recited in claim 1 or claim 2, **characterized in that**, comprising or consisting of an amino acid sequence as set forth in SEQ ID NO: 3.

5. A nucleic acid molecule, **characterized in that**, comprising a nucleotide sequence which encodes a GPCR chimeric opsin of any one of claim 1 to claim 4.

6. The nucleic acid molecule, as recited in claim 5, comprising or consisting of a nucleotide sequence as set forth in SEQ ID NO: 4.

7. A vector, preferably an AAV vector, **characterized in that**, the vector comprises a nucleotide sequence which encodes the GPCR chimeric opsin of any one of claim 1 to claim 4, or comprises the nucleic acid molecule of claim 5 or claim 6.

8. A transgenic cell, preferably a transgenic retinal ganglion cell, **characterized in that**, comprising the GPCR chimeric opsin of any one of claim 1 to claim 4, the nucleic acid molecule of claim 5 or claim 6, or the vector of claim 7.

9. Use of the GPCR chimeric opsin of any one of claim 1 to claim 4, the nucleic acid molecule of claim 5 or claim 6, the vector of claim 7, or the transgenic cell of claim 8, for the manufacture of a medicament for improving vision in a subject in need thereof.

10. Use of the GPCR chimeric opsin of any one of claim 1 to claim 4, the nucleic acid molecule of claim 5 or claim 6, the vector of claim 7, or the transgenic cell of claim 8, for the manufacture of a medicament for treating a retinal neurodegenerative disease in a subject in need thereof, preferably, the retinal neurodegenerative disease is retinitis pigmentosa (RP) or age-related macular degeneration (AMD).
